(19) Europäisches Patentamt / European Patent Office / Office européen des brevets

(11) **EP 2 621 930 B1**

(12) **FASCICULE DE BREVET EUROPEEN**

(45) Date de publication et mention
de la délivrance du brevet:
**02.09.2015 Bulletin 2015/36**

(21) Numéro de dépôt: **11779772.0**

(22) Date de dépôt: **30.09.2011**

(51) Int Cl.:
**C07D 493/04** (2006.01)     **C07H 3/10** (2006.01)
**B01J 2/02** (2006.01)       **B01J 2/30** (2006.01)

(86) Numéro de dépôt international:
**PCT/FR2011/052289**

(87) Numéro de publication internationale:
**WO 2012/042187 (05.04.2012 Gazette 2012/14)**

(54) **PASTILLES DE DIANHYDROHEXITOLS DE GRANDE FLUIDITE ET NON MOTTANTES**

DIANHYDROHEXITOL-PELLETS MIT HOHER FLIESSFÄHIGKEIT UND OHNE KLUMPENBILDUNG

HIGH-FLUIDITY NON-CAKING DIANHYDROHEXITOL PELLETS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.09.2010 FR 1057949**

(43) Date de publication de la demande:
**07.08.2013 Bulletin 2013/32**

(73) Titulaire: **Roquette Frères
62136 Lestrem (FR)**

(72) Inventeurs:
• **IBERT, Mathias
F-59930 La Chapelle D'armentieres (FR)**

• **SAINT POL, Jérome
62122 LAPUGNOY (FR)**
• **WYART, Hervé
F-62149 Cuinchy (FR)**

(74) Mandataire: **Cabinet Plasseraud
52, rue de la Victoire
75440 Paris Cedex 09 (FR)**

(56) Documents cités:
**WO-A1-03/043959     WO-A2-2009/010673
JP-A- 2006 117 649**

**Description**

DOMAINE DE L'INVENTION

La présente invention est relative à des pastilles de dianhydrohexitols dont la fluidité est préservée même après un stockage de longue durée. Ces pastilles de dianhydrohexitols présentent ainsi la particularité de ne pas être sujettes au mottage.

ART ANTERIEUR

Les dianhydrohexitols (1,4 - 3,6-dianhydro-hexitols), également appelés isohexides, sont des produits de déshydratation interne de sucres hydrogénés en $C_6$ (hexitols) tels que le sorbitol, le mannitol et l'iditol. Dans la présente demande, le terme « dianhydrohexitols » englobe l'isosorbide (1,4 - 3,6-dianhydro-sorbitol), l'isomannide (1,4 - 3,6 dianhydro-mannitol), l'isoidide (1,4 - 3,6-dianhydro-iditol) et les mélanges d'au moins deux de ces produits.

Actuellement, il existe un fort développement des applications industrielles des dianhydrohexitols, notamment dans le domaine pharmaceutique, dans ceux des intermédiaires de synthèse chimique et des matières plastiques.

Pour la majorité de ces applications, il est généralement nécessaire de disposer de compositions les plus pures possible, ayant notamment une teneur en dianhydrohexitols au moins égale à 98,5 % en poids, de préférence au moins égale à 99,5 % en poids desdites compositions sur sec.

Les dianhydrohexitols, et en particulier l'isosorbide, sont des produits fortement hygroscopiques, chimiquement peu stables et, par conséquent, fortement sujets au mottage.

La Demanderesse a en particulier observé que le stockage de dianhydrohexitols fabriqués selon des procédés connus, entraînait dans certaines conditions d'humidité et de température une dégradation chimique et un mottage important.

Les compositions de dianhydrohexitols ainsi mottées lors du stockage soulèvent bon nombre de problèmes. En effet, le mottage induit non seulement de sérieuses difficultés de manipulation lors d'opérations telles que leur transfert, leur déballage, leur broyage, leur remise en solution, etc mais il impacte également fortement sur le rendement de ces opérations.

Un certain nombre de solutions a été proposé pour remédier à ces difficultés :

- ajouter à la composition de dianhydrohexitols des agents antimottants,
- élaborer un matériau d'emballage externe particulier tel qu'il prévient la reprise en eau de la composition et, par conséquence, le mottage de ladite composition.

Ainsi, dans le domaine plus général des polyols, le brevet JP n° 74 88 183 divulgue l'addition d'esters d'acides organiques ou d'acétals comme agents antimottants. Selon ce brevet, l'addition de 0,005% en poids d'acétate de butylcellulose permet au néopentylglycol de ne pas présenter de mottage pendant 30 jours sous une pression de 0,23 bar après sa mise en forme.

Un autre procédé pour empêcher les polyols de motter a également été décrit dans le brevet FR n° 2 477 902. Il a ainsi été proposé l'addition de 0,005% à 0,25% en poids d'amines tertiaires qui contiennent au moins deux substituants organiques identiques afin d'empêcher le mottage des polyols.

Cependant, l'addition d'agents antimottants n'est généralement pas retenue par les spécialistes du domaine spécifique des compositions de dianhydrohexitols, notamment car :

- l'introduction d'impuretés dans lesdites compositions peut nuire à leurs propriétés et donc à leur valeur commerciale,
- les contraintes réglementaires interdisent l'utilisation de tels additifs dans certaines applications.

Quant à la mise en oeuvre d'un procédé d'emballage des compositions de dianhydrohexitols, cette seconde solution technique n'a jusqu'alors pas donné de résultats entièrement satisfaisants.

Parmi ces procédés d'emballage, la demande de brevet JP 2006-117649 divulgue l'utilisation d'un matériau d'emballage de type film pour emballer de l'isosorbide dans le but de préserver celui-ci contre l'absorption d'eau, de le maintenir sous forme de poudre fluide et d'empêcher la formation d'agrégats. Le film d'emballage est défini comme étant un film multicouche à base de matières plastiques et d'aluminium.

La Demanderesse elle-même a élaboré un emballage à base de polymère thermoplastique pour emballer des dianhydrohexitols. Ledit emballage, divulgué dans la demande de brevet FR n° 2919587, est destiné aux compositions tant solides que liquides de dianhydrohexitols. En tant que formes solides, il peut s'agir, par exemple, de distillats refroidis et solidifiés ou de cristaux, l'ensemble de ces produits pouvant notamment se présenter sous forme d'une poudre ou d'écailles. Cependant, ledit emballage a donné de très bons résultats en termes de prévention de la dégradation chimique des compositions de dianhydrohexitols lors du stockage mais n'a pas permis d'empêcher durablement le mottage desdites compositions.

La présente invention a donc pour but de fournir des compositions de dianhydrohexitols dont la fluidité est préservée même après un stockage de plusieurs centaines de kilogrammes de produit empilées durant de longues durées.

Un autre but particulier de la présente invention est également de fournir des compositions de dianhydrohexitols pouvant s'écouler facilement, faciles à doser en volume et ne laissant pas ou presque de résidus dans l'emballage vidé après usage.

Un but particulier de la présente invention est de fournir des compositions ne formant pas ou peu de poussières.

Un autre but de la présente invention est de fournir des compositions mettant en oeuvre de très faibles quantités d'agent antimottant et, avantageusement, n'en mettant pas en oeuvre du tout.

Un autre but de la présente invention est de fournir des compositions de teneur extrêmement élevée en dianhydrohexitols, notamment en isosorbide, i.e. contenant entre 90% et 100%, de préférence entre 95% et 100% et, plus préférentiellement encore entre 97% et 100% en poids sur sec de dianhydrohexitols.

Un autre but de la présente invention est de fournir des compositions de dianhydrohexitols pouvant présenter une humidité résiduelle relativement importante, par exemple de l'ordre de 0,2% à 0,5% en poids desdites compositions, et présentant pourtant une faible propension au mottage.

Un autre but encore de la présente invention est de fournir des compositions de dianhydrohexitols aptes à être solubilisées ou fondues rapidement.

## RESUME DE L'INVENTION

La présente invention a pour objet des pastilles de dianhydrohexitols comprenant entre 90% et 100%, de préférence entre 95% et 100%, et plus préférentiellement entre 97% et 100% en poids sur sec de la composition desdites pastilles de dianhydrohexitols et dont au moins 90% en poids desdites pastilles présentent une granulométrie sur tamis supérieure ou égale à 2000 $\mu$m.

**[0023]** - Les pastilles de dianhydrohexitols selon l'invention comprennent moins de 2% d'agent anti-mottant, préférentiellement moins de 0,5%, plus préférentiellement encore moins de 0,01% et, encore plus préférentiellement, moins de 0,001% en poids sur sec de la composition desdites pastilles de dianhydrohexitols. Avantageusement, les pastilles selon l'invention sont exemptes de tout agent anti-mottant et sont pourtant peu sujettes au mottage.

L'invention a également pour objet un procédé de préparation desdites pastilles de dianhydrohexitols.

## DESCRIPTION DETAILLEE

La présente invention a pour objet des pastilles de dianhydrohexitols comprenant entre 90% et 100%, de préférence entre 95% et 100%, et plus préférentiellement entre 97% et 100% en poids (sec/sec) de la composition desdites pastilles de dianhydrohexitols et dont au moins 90% en poids desdites pastilles présentent une granulométrie sur tamis supérieure ou égale à 2000 $\mu$m.

Plus préférentiellement encore, les pastilles selon l'invention présentent une teneur en dianhydrohexitols supérieure ou égale à 98,5 % en poids, préférentiellement supérieure ou égale à 99,5% en poids (sec/sec) de la composition desdites pastilles de dianhydrohexitols. Le complément à 100% en poids sur sec de la composition desdites pastilles de dianhydrohexitols peut être constitué d'hexitols, de monoanhydrohexitols, d'agents stabilisants tels que ceux cités dans le brevet EP 1446373, d'agents anti-mottants, d'impuretés et co-produits divers tels que ceux cités dans le brevet EP 1287000 (paragraphe [0008]) et liés au procédé de production des dianhydrohexitols, notamment à l'étape de déshydratation dudit procédé.

Les pastilles selon l'invention contiennent en outre peu ou ne contiennent pas d'agent anti-mottant et sont pourtant peu sujettes au mottage.

L'invention a également pour objet un procédé de préparation desdites pastilles de dianhydrohexitols.

Dans la présente demande, on entend par « pastille » un produit compact en trois dimensions obtenu par « pastillage ». Ainsi, le terme « pastille » comprend les termes comprimé, galet, granule, tablette, bille et/ou toute autre forme obtenue par « pastillage ». Ladite pastille peut présenter des faces supérieure et inférieure plates ou bombées, concaves ou convexes. Ladite pastille peut être indifféremment de forme générale ronde, ovale, carrée, rectangulaire, octogonale, polygonale, etc. De préférence, la pastille selon l'invention est en forme de dôme, c'est-à-dire qu'elle possède une face plane et une face convexe, les arêtes entre les deux faces étant plus ou moins adoucies.

Dans la présente demande, on entend par « pastillage » un procédé combinant une étape de dépose de gouttes à une étape de refroidissement de ladite goutte, ledit procédé permettant ainsi de produire des pastilles aux formes stables et uniformes, substantiellement exemptes de particules de fine granulométrie. Dans la présente demande, on entend par « goutte » toute quantité définie de produit fondu. Ainsi, les pastilles selon l'invention sont solidifiées directement à partir de dianhydrohexitols fondus, éliminant ainsi des frais d'énergie et d'équipements associés à des étapes ultérieures de broyage, de concassage ou à toutes autres étapes du même type.

Les pastilles selon l'invention peuvent être obtenues en mettant en oeuvre différents types de pastilleuses, telles que des pastilleuses à disques, des pastilleuses sur bandes, etc. En particulier, le pastillage selon l'invention peut être mis en oeuvre selon les procédés de pastillage décrits dans la demande de brevet WO 2009/010673.

Préalablement au procédé de pastillage, la composition de dianhydrohexitols est maintenue à l'état fondu dans une cuve d'alimentation. On parle alors de « fondu » de dianhydrohexitols. Pour ce faire, le(les) dianhydrohexitol(s) est(sont) avantageusement maintenu(s) à une température supérieure ou égale à sa(leur) température de fusion, en particulier, pour l'isosorbide, à une température supérieure ou égale à 63 ± 2°C. La composition de dianhydrohexitols peut-être issue d'une distillation d'un brut réactionnel, d'une fusion d'un produit purifié par cristallisation en phase fondue ou en solvant aqueux ou organique, d'une concentration à sec d'une solution de dianhydrohexitols purifiée selon le brevet EP 1 287 000.

De manière préférée, le fondu est introduit dans l'unité de pastillage via une tuyauterie chauffée et une pompe à débit constant. La pompe réglable permet l'alimentation de la pastilleuse à la pression requise.

La première étape du procédé de pastillage consiste en la production de gouttes de dianhydrohexitols à partir d'un fondu. Selon un mode préféré de l'invention, le fondu arrive à une unité d'alimentation-dosage où il est transformé en pastilles, dont la granulométrie et la quantité sont déterminées par le diamètre et le nombre de trous ou buses sélectionnés. Il est injecté dans un ou plusieurs générateurs de gouttes, par exemple par surpression, qui transforment la coulée de produit continue en gouttes uniformes de diamètre déterminé.

L'étape de production de gouttes de dianhydrohexitols est suivie d'une étape de refroidissement desdites gouttes. Cette étape de refroidissement peut être effectuée par tout type de procédé bien connu de l'homme du métier, par exemple par immersion dans un réfrigérant (trempe, air froid, etc) ou par dépôt de gouttes sur une surface métallique en mouvement refroidie par un liquide réfrigérant. Préférentiellement, cette étape de refroidissement est réalisée par dépôt de gouttes sur une bande de refroidissement constituée d'une bande métallique en mouvement refroidie par un circuit d'eau ou par dépôt des gouttes sur un plateau horizontal refroidi par un liquide réfrigérant. Le refroidissement des gouttes doit être tel que lesdites gouttes doivent atteindre une température inférieure à la température de fusion du(des) dianhydrohexitol(s), en particulier inférieure à 63 ± 2°C pour l'isosorbide, de telle sorte que la forme desdites pastilles soit stabilisée définitivement.

La synchronisation de l'unité d'alimentation-dosage et du système de refroidissement des disques refroidisseurs, qui s'effectue lors du dosage des gouttes de fondu, assure le façonnage des pastilles. En outre, l'appareil peut être équipé d'un système de refroidissement en circuit fermé pour éviter toute contamination du produit par les vapeurs d'eau ou par le fluide réfrigérant, de même que toute contamination du fluide réfrigérant.

Selon un mode préféré de la présente invention, la pastilleuse peut être une pastilleuse ROTOFORM® commercialisée par la société SANDVIK. Une telle pastilleuse est constituée d'un corps cylindrique fixe (stator) chauffé comportant une rainure d'alimentation longitudinale et autour duquel se trouve un tube perforé en rotation. Lorsqu'une série de perforations du tube passe sous la rainure du stator, une petite quantité de produit est libérée et déposée sous forme de gouttes sur la bande en acier où elles sont refroidies et solidifiées. La vitesse périphérique de la pastilleuse est synchronisée avec la vitesse de défilement de la bande d'acier.

Préalablement au procédé de pastillage, la composition de dianhydrohexitols est préparée de quelque manière que ce soit, par exemple selon le procédé décrit dans le brevet EP 1 287 000, elle peut être soumise à divers procédés de purification, concentration, cristallisation, etc, bien connus de l'homme du métier. En particulier, les dianhydrohexitols peuvent être soumis à au moins un traitement avec du charbon actif et/ou au moins un moyen d'échange d'ions. Par ailleurs, les dianhydrohexitols peuvent être préalablement stabilisés selon l'enseignement du brevet EP 1 446 373.

Les pastilles de dianhydrohexitols selon l'invention comprennent entre 90% et 100%, de préférence entre 95% et 100%, et plus préférentiellement entre 97% et 100% en poids sur sec de la composition desdites pastilles de dianhydrohexitols.

Lesdites pastilles selon l'invention comprennent en outre moins de 2% d'agent anti-mottant, préférentiellement moins de 0,5%, plus préférentiellement encore moins de 0,01% et encore plus préférentiellement moins de 0,001% en poids sur sec de la composition desdites pastilles de dianhydrohexitols.

Les pastilles conformes à l'invention sont par ailleurs caractérisées par leur masse volumique aérée et leur masse volumique tassée ainsi que par leur tassement, les valeurs étant calculées selon le test A ci-après décrit, grâce à l'appareil STAMPF VOLUMETER STAV 2003.

Dans ces conditions, les pastilles conformes à l'invention présentent avantageusement

- une masse volumique aérée comprise entre 0,80 et 1,00 g/ml, de préférence comprise entre 0,81 et 0,85 g/ml, encore plus préférentiellement comprise entre 0,82 et 0,84 g/ml,
- une masse volumique tassée comprise entre 0,81 et 1,00 g/ml, de préférence comprise entre 0,82 et 0,86 g/ml, encore plus préférentiellement comprise entre 0,83 et 0,85 g/ml, et

- un tassement au plus égal à 2%, de préférence compris entre 0,5% et 2%, encore plus préférentiellement compris entre 0,8% et 1,6%.

Les valeurs de masse volumique tassée et aérée ainsi que de tassement des pastilles conformes à l'invention sont déterminées, selon le test A, en utilisant l'appareil STAMPF VOLUMETER STAV 2003, en suivant la méthode préconisée dans le mode d'emploi dudit STAMPF VOLUMETER. Ainsi, le test A consiste à introduire une quantité de produit telle qu'elle emplisse un volume de 250 ml dans une éprouvette de diamètre 35 mm et de hauteur 335 mm. Quel que soit le produit testé, le produit est introduit dans ladite éprouvette de manière à remplir toujours un même volume de 250 ml (volume avant tassement). Le volume de produit (volume après tassement) est ensuite mesuré après 1250 coups donnés du haut vers le bas (descente de 3mm +/- 0,2).

L'appareil STAMPF VOLUMETER STAV 2003 permet ainsi de mesurer, dans des conditions standardisées et reproductibles, l'aptitude au tassement d'un produit en calculant la masse volumique aérée, la masse volumique tassée et, à partir de ces données, les valeurs de tassement selon les formules suivantes :

```
Masse volumique aérée = masse de produit introduit dans
l'éprouvette (g) / 250 (ml)
```

```
Masse volumique tassée = masse de produit introduit dans
l'éprouvette (g) / X (ml)
```

avec X = volume (ml) occupé par le produit après tassement

```
Tassement(%)= [(masse volumique tassée - masse volumique
aérée) / masse volumique aérée] x 100
```

avec masse volumique tassée = masse volumique tassée après un tassement réalisé par 1250 coups donnés du haut vers le bas (descente de 3,0 ± 0,2 mm).

Les pastilles de dianhydrohexitols conformes à l'invention peuvent être également caractérisées par leur compressibilité, évaluée selon le test B ci-après décrit.

Le test de compression B consiste à introduire une certaine masse de produit, quantifiée en grammes, dans un cylindre creux en laiton de 4,8 cm de diamètre intérieur et 8 cm de hauteur, disposé dans un cristallisoir de diamètre 95 mm. Un piston de 1,3 kg, s'emboîtant exactement dans le cylindre creux en laiton, est posé sur le produit contenu dans ledit cylindre. Quelque soit le produit testé, le cylindre comprend toujours sensiblement le même volume de produit (la hauteur de remplissage du cylindre étant sensiblement la même, fixée à 5,9 ± 0,2 cm). Immédiatement après la mise en place du piston sur le produit introduit dans le cylindre, la hauteur précise de l'intégralité du produit introduit dans le cylindre est mesurée (hauteur avant compression). L'ensemble piston / produit / cylindre / cristallisoir est ensuite placé dans un sachet (22 cm x 41 cm) en aluminium (sachet Z 183407 commercialisé par la société ALDRICH). Le sachet est immédiatement fermé par soudage à l'aide d'une soudeuse thermique à impulsions (modèle SZ 380 commercialisé par la société Joisten & Kettenbaum GmbH & Co, Bergisch Gladbach, Allemagne) afin d'assurer l'étanchéité vis-à-vis de l'atmosphère extérieure. Les échantillons ainsi emballés sont placés pendant 1 semaine dans une étuve ventilée, thermostatée à une température soit de 20 °C, soit de 40°C. Après cette période d'une semaine, le sachet est ouvert et la hauteur précise de l'intégralité du produit introduit dans le cylindre est mesurée (hauteur après compression). La compressibilité du produit est calculée selon les formules suivantes :

```
M_avant = masse de produit introduit dans le cylindre (g) / [(∏
x d² x h_avant)/4]
```

avec $M_{avant}$ = masse volumique avant compression en g/cm³ ; d = diamètre intérieur du cylindre (cm) = 4,8 cm ; havant = hauteur du produit dans le cylindre avant compression (cm) ; $\Pi$ = pi

```
M_après = masse de produit introduit dans l'éprouvette
(g) / [(∏ x d² x h_après)/4]
```

avec $M_{après}$ = masse volumique après compression en g/cm$^3$, d = diamètre intérieur du cylindre (cm) = 4,8 cm, $h_{après}$ = hauteur du produit dans le cylindre après compression (cm)

$$Compressibilité(\%) = [(M_{après} - M_{avant}) / M_{avant}] \times 100$$

Les pastilles conformes à l'invention présentent avantageusement une compressibilité, évaluée à 20°C, inférieure à 5%, préférentiellement inférieure à 3%, plus préférentiellement encore inférieure à 2%. Lesdites pastilles présentent en outre avantageusement une compressibilité, évaluée à 40°C, inférieure à 5%, préférentiellement inférieure à 4,5%.

Les pastilles conformes à l'invention peuvent également être caractérisées par leur faible aptitude au mottage. L'aptitude au mottage est évaluée, en particulier, grâce au test C ci-après décrit.

Le test de mottage C, similaire à celui décrit dans la première revendication de la demande de brevet EP 1 787 993 A1, consiste à introduire 70 g de produit dans un flacon en verre de 12 cm de hauteur et de 6 cm de diamètre intérieur. Ledit flacon ainsi rempli est refermé hermétiquement et placé, durant 2 ou 4 semaines, dans une étuve ventilée dont la température intérieure est fixée à 20°C ou à 40°C. Après 2 ou 4 semaines, le flacon est retiré de l'étuve, ouvert puis retourné d'un angle de 90° puis de 180° selon un axe horizontal. Un score est attribué au produit selon son mode d'écoulement :

- un score de 0 est attribué au produit s'il s'écoule complètement et immédiatement quand le flacon est placé à 90° ;
- un score de 1 est attribué au produit s'il s'écoule complètement dans une durée maximale de 1 minute quand le flacon est placé à 180° ;
- un score de 2 est attribué au produit s'il

    o ne s'écoule pas, même après que le flacon ait été placé à 180° durant plus d'une minute, mais
    o s'écoule après que l'on ait fait chuter, sur le culot du flacon placé à 180°C, une masse de 300g d'une hauteur de 10 cm ;

- un score de 3 est attribué au produit s'il ne s'écoule pas, même après que le flacon ait été placé à 180° durant plus d'une minute et que l'on ait fait chuter, sur le culot du flacon placé à 180°C, une masse de 300 g d'une hauteur de 10 cm.

Les pastilles conformes à l'invention présentent avantageusement un score de 1 au test de mottage C réalisé à 20°C durant 2 ou 4 semaines et un score de 1 ou 2 au test de mottage C réalisé à 40°C durant 2 ou 4 semaines.

Les pastilles conformes à l'invention peuvent également présenter l'avantage d'être chimiquement stables durant le stockage. Selon l'invention, la stabilité chimique est évaluée, selon le test D, par pHmétrie (vérification de la stabilité du pH).

Le test de stabilité D consiste à évaluer dans un premier temps le pH d'un échantillon de produit dissous à 40% en poids de matière sèche dans de l'eau osmosée. On introduit ensuite 50 g d'un autre échantillon de ce même produit dans un flacon en verre puis on referme hermétiquement ledit flacon et on le place dans une étuve ventilée, thermostatée à une température de 50 °C. Plusieurs flacons, remplis du même produit, sont placés dans l'étuve. Après une période déterminée, la totalité de l'échantillon de produit est extrait des flacons et dissous à 40 % en poids de matière sèche dans de l'eau osmosée. La mesure de pH s'effectue sur un pHmètre de marque RADIOMETER ANALYTICAL PHM 220 équipé d'une électrode combinée à fil Ag/AgCl de marque METTLER TOLEDO, préalablement étalonnée à l'aide de solutions tampon pH 7 et 4.

Les pastilles conformes à l'invention présentent avantageusement un pH, évalué selon le test D, supérieur ou égal à 7, préférentiellement compris entre 7,0 et 8,5, après 6 mois de stockage dans un flacon de verre placé dans une étuve ventilée, thermostatée à une température de 50 °C. Ce pH supérieur ou égal à 7 montre une absence de génération d'acidité (synonyme de dégradation des dianhydrohexitols avec formation d'acide formique) et montre une excellente stabilité des pastilles de dianhydrohexitols conformes à l'invention.

Les pastilles conformes à l'invention présentent également l'avantage de se solubiliser rapidement, leur durée de solubilisation étant la même que celles des autres mises en forme connues jusqu'alors. Ainsi, les pastilles conformes à l'invention peuvent être caractérisées par une durée de solubilisation dans l'eau inférieure ou égale à

- 12 minutes, préférentiellement 10 minutes, lorsque lesdites pastilles sont solubilisées à 20°C à une matière sèche finale dans la solution de 50% ; et
- 6 minutes, préférentiellement 5 minutes, lorsque lesdites pastilles sont solubilisées à 40°C à une matière sèche finale dans la solution de 50%.

Selon l'invention, la solubilisation est évaluée, selon le test E. Ledit test de solubilisation E consiste à introduire une prise d'essai de 100 g de produit dans un bécher de 250 ml contenant 100 ml d'eau distillée préalablement chauffée à 20°C ± 2°C ou à 40°C ± 2°C. L'ensemble produit / eau distillée est agité grâce à un barreau aimanté (référence N°ECN 442-4510 / société VWR). On détermine alors le temps écoulé entre l'introduction de la prise d'essai dans le bécher et la solubilisation complète de la prise d'essai dans l'eau. L'expérimentation est réalisée 3 fois pour chaque échantillon. Selon la présente invention, la durée de solubilisation de l'échantillon correspond à la moyenne des résultats de trois expérimentations.

Les pastilles de dianhydrohexitols conformes à l'invention présentent également l'avantage de pouvoir fondre rapidement, leur durée de fusion étant du même ordre de grandeur que celles des autres mises en forme connues jusqu'alors. Ainsi, les pastilles conformes à l'invention peuvent être caractérisées par une durée de fusion inférieure à 25 minutes, préférentiellement 22 minutes, lorsque lesdites pastilles sont chauffées à 80°C.

Selon l'invention, la durée de fusion est évaluée, selon le test F. Ledit test de fusion F consiste à introduire une prise d'essai de 100 g de produit dans un bécher de 250 ml préalablement chauffé à 80°C ± 2°C. La prise d'essai est agitée grâce à un barreau aimanté (référence N°ECN 442-4510 / société VWR). On détermine alors le temps écoulé entre l'introduction de la prise d'essai dans le bécher et la fonte complète de la prise d'essai. L'expérimentation est réalisée 3 fois pour chaque échantillon. Selon la présente invention, la durée de fusion de l'échantillon correspond à la moyenne des résultats de trois expérimentations.

Les pastilles conformes à l'invention sont également caractérisées en ce qu'au moins 90 %, préférentiellement 95%, plus préférentiellement encore 94% en poids, des pastilles présentent une granulométrie sur tamis supérieure ou égale à 2000 µm, préférentiellement comprise entre 2000 µm et 20000 µm. Selon l'invention, la granulométrie sur tamis des pastilles est évaluée selon le test G ci-après décrit.

Ledit test G est réalisé en utilisant la tamiseuse de laboratoire VS 1000 commercialisée par la société RETSCH, en suivant la méthode préconisée dans le mode d'emploi de ladite tamiseuse. Selon le test G, ladite tamiseuse est équipée d'une tour de tamisage constituée de 7 tamis de 20 cm de diamètre dont la maille est respectivement de 20000 µm, 5000 µm, 2000 µm, 1400 µm, 1000 µm, 500 µm, et 355 µm (les tamis sont placés de haut en bas, de la maille la plus large jusqu'à la maille la plus étroite). Brièvement, le test G consiste à introduire une prise d'essai de 200 g de produit au sommet de la tour de tamisage et de démarrer la tamiseuse en mode continu, à une amplitude de vibration de 50%, durant 10 minutes. Après un tamisage de 10 minutes, la tamiseuse est arrêtée et la quantité de produit retenue sur chacun des tamis est quantifiée par pesée.

Les pastilles selon l'invention présentent préférentiellement une teneur en humidité, évaluée par la méthode de Karl Fisher, inférieure à 1%, préférentiellement inférieure à 0,5%, plus préférentiellement encore inférieure à 0,3% en poids de la composition de dianhydrohexitols desdites pastilles.

Les pastilles selon l'invention ou susceptibles d'être obtenues par le procédé selon l'invention permettent de réaliser des compositions particulières adaptées à des domaines aussi diversifiés que la nutraceutique, la pharmacie, la cosmétique, la chimie, les matériaux de construction, les papiers-cartons, les polymères. Ainsi, la présente invention concerne en outre l'utilisation des pastilles selon l'invention ou obtenues par le procédé selon l'invention pour la fabrication de dérivés de dianhydrohexitols et de polymères contenant au moins un dianhydrohexitol ou un dérivé de celui-ci.

L'invention sera encore mieux comprise à l'aide des exemples qui suivent, lesquels ne se veulent pas limitatifs et font seulement état de certains modes de réalisation et de certaines propriétés avantageuses des pastilles conformes l'invention.

EXEMPLES

Dans les exemples 2 à 6 ci-après, on entend par :

- « pastilles V1, V2 et V3 » des pastilles d'isosorbide, conformes à l'invention, d'épaisseur 2 mm et dont la plus grande longueur (diamètre) est respectivement de 4 mm, 5 mm et 7 mm, obtenues selon le procédé de pastillage détaillé dans l'exemple 1 ;

- « écailles » des écailles d'isosorbide obtenues par cristallisation sur un cylindre refroidi en rotation, l'alimentation se faisant par trempage dans un bac contenant l'isosorbide fondu.

- « cristaux vrac » des cristaux d'isosorbide obtenus par cristallisation dans un solvant de type alcool, suivie d'une filtration et séchage sous vide dans un filtre sécheur desdits cristaux obtenus.

- « cristaux 355-1400 » des cristaux issus d'une coupe granulométrique 355-1400 µm réalisée à partir des « cristaux vrac » ci-dessus décrits. La coupe granulométrique est effectuée en utilisant la tamiseuse de laboratoire VS 1000 commercialisée par la société RETSCH, en suivant la méthode préconisée dans le mode d'emploi de ladite tamiseuse. Ladite tamiseuse est équipée pour effectuer la coupe d'une tour de tamisage constituée de 2 tamis de 20 cm de diamètre dont la maille est respectivement de 355 µm et 1400 µm (les tamis sont placés de haut en bas, de la maille la plus large jusqu'à la maille la plus étroite).

Exemple 1 : Préparation de pastilles d'isosorbide conformes à l'invention

Dans une cuve cylindrique chauffée et agitée, on introduit de l'isosorbide de pureté 99,8% et contenant 0,2% d'eau sous forme d'écailles. Lorsque le produit est totalement fondu, on le maintient à une température de 65°C ± 2°C et on procède au pastillage à l'aide de la pastilleuse Rotoform® 3000 commercialisée par la société SANDVIK.

Paramètres de fonctionnement :

- Température d'alimentation : 65°C ± 2°C
- Vitesse de la bande : 9,5 m/min
- Vitesse du Rotoform® : 11 m/mn
- Largeur de bande : 600 mm
- Longueur de bande froide : 7,5 m
- Longueur totale de la bande : 10 m
- Eau de refroidissement de la bande : eau décarbonatée filtrée à 20°C avec un débit de 5 m$^3$/h
- Débit de production des pastilles : 360 kg/h

Le choix du diamètre de perforation du tube d'alimentation permet d'obtenir des pastilles de différents diamètres.

On obtient les pastilles suivantes en appliquant les paramètres de fonctionnement décrits ci-dessus avec des tubes d'alimentation de diamètres de perforation adéquats :

- pastilles V1 : diamètre 4 mm et épaisseur 2 mm
- pastilles V2 : diamètre 5 mm et épaisseur 2 mm
- pastilles V3 : diamètre 7 mm et épaisseur 2 mm

De même, on procède de la même manière que précédemment en prenant comme produit de départ de l'isosorbide de pureté 97% sous forme d'écailles. On obtient ainsi les pastilles suivantes en appliquant les paramètres de fonctionnement décrits ci-avant avec des tubes d'alimentation de diamètres de perforation adéquats :

- pastilles V4 : diamètre 4 mm et épaisseur 2 mm
- pastilles V5 : diamètre 5 mm et épaisseur 2 mm
- pastilles V6 : diamètre 7 mm et épaisseur 2 mm

Exemple 2 : Comparaison du comportement des compositions d'isosorbide conformes à l'invention et des compositions d'isosorbide sous forme d'écailles ou de cristaux lors de tests de tassement et de compression

**2.1 Test de tassement**

La masse volumique aérée, la masse volumique tassée et le tassement de différentes mises en forme d'isosorbide sont évaluées selon le test A. Ces paramètres sont en particulier comparés pour des pastilles d'isosorbide de différents diamètres (V1, V2 et V3), conformes à l'invention, des écailles d'isosorbide et des cristaux d'isosorbide (cristaux vrac ou issus d'une coupe 355-1400 µm).

Les résultats de cette évaluation sont présentés dans le tableau 1.

Tableau 1 : Résultats des tests de tassement effectués sur différentes mises en forme d'isosorbide

| Forme | Volume après tassement (ml) | Masse (g) | Masse volumique avant tassement (g/ml) | Masse volumique après tassement (g/ml) | Tassement (%) |
|---|---|---|---|---|---|
| Pastilles V1 | 248 | 207,7 | 0,83 | 0,84 | 0,8 |
| Pastilles V2 | 246 | 208,9 | 0,84 | 0,85 | 1,6 |
| Pastilles V3 | 248 | 205,3 | 0,82 | 0,83 | 0,9 |
| Ecailles | 239 | 185,9 | 0,74 | 0,78 | 4,6 |
| Cristaux vrac | 230 | 168,1 | 0,67 | 0,73 | 8,8 |

(suite)

| Forme | Volume après tassement (ml) | Masse (g) | Masse volumique avant tassement (g/ml) | Masse volumique après tassement (g/ml) | Tassement (%) |
|---|---|---|---|---|---|
| Cristaux (355-1400 $\mu$m) | 230 | 166,7 | 0,67 | 0,73 | 8,7 |

Les pastilles conformes à l'invention présentent une masse volumique aérée et une masse volumique tassée très supérieures à celles des produits existant sur le marché (écailles et cristaux). Lesdites pastilles présentent en outre un tassement très nettement inférieur à celui des écailles et des cristaux.

Par rapport aux autres modes de mise en forme, les pastilles d'isosorbide sont donc plus aptes à être transportées en gros volume puisqu'elles se tassent moins et sont donc moins susceptibles au mottage et qu'elles présentent malgré cela une masse volumique tant tassée qu'aérée plus élevée.

**2.2 Test de compression**

La masse volumique avant compression ($M_{avant}$), la masse volumique après compression ($M_{après}$) et la compressibilité de différentes mises en forme d'isosorbide sont évaluées selon le test B. Ces paramètres sont en particulier comparés pour des pastilles d'isosorbide V1, V2 et V3, conformes à l'invention, des écailles d'isosorbide et des cristaux d'isosorbide (cristaux vrac ou issus d'une coupe 355-1400 $\mu$m).

Les résultats de cette évaluation sont présentés dans le tableau 2.

Tableau 2 : Résultats des tests de compression effectués sur différentes mises en forme d'isosorbide

| Etuve thermostatée à 20°C: | | | | | | |
|---|---|---|---|---|---|---|
| Forme | Hauteur avant (cm) | Hauteur après (cm) | Masse avant (g) | $M_{avant}$ (g/cm$^3$) | $M_{après}$ (g/cm$^3$) | Compressibilité (%) |
| Pastilles V1 | 5,8 | 5, 7 | 90,5 | 0,86 | 0,88 | 2 |
| Pastilles V2 | 5, 7 | 5, 7 | 90,5 | 0,88 | 0,88 | 0 |
| Pastilles V3 | 5,9 | 5,9 | 91,1 | 0, 85 | 0, 85 | 0 |
| Ecailles | 6,1 | 5, 7 | 83,6 | 0, 76 | 0,81 | 7 |
| Cristaux Vrac | 5,9 | 5,3 | 78,4 | 0, 73 | 0,82 | 11 |
| Cristaux 355-1400 | 5,9 | 5,3 | 78,6 | 0, 74 | 0,82 | 11 |
| Etuve thermostatée à 40°C: | | | | | | |
| Forme | Hauteur (cm) avant tassement | Hauteur (cm) après tassement | Masse (g) avant tassement | $M_{avant}$ (g/cm$^3$) | $M_{après}$ (g/cm$^3$) | Compressibilité (%) |
| Pastilles V1 | 5,8 | 5,6 | 90,6 | 0,86 | 0,89 | 4 |
| Pastilles V2 | 5,7 | 5,5 | 90,6 | 0,88 | 0,91 | 4 |
| Pastilles V3 | 6,0 | 5,8 | 92,4 | 0,85 | 0,88 | 4 |
| Ecailles | 5,9 | 5,5 | 83,7 | 0,78 | 0,84 | 7 |

(suite)

**Etuve thermostatée à 40°C:**

| Forme | Hauteur (cm) avant tassement | Hauteur (cm) après tassement | Masse (g) avant tassement | $M_{avant}$ (g/cm$^3$) | $M_{après}$ (g/cm$^3$) | Compressibilité (%) |
|---|---|---|---|---|---|---|
| Cristaux Vrac | 5,8 | 5,1 | 77,4 | 0,74 | 0,84 | 14 |
| Cristaux 355-1400 | 5,8 | 5,1 | 78,5 | 0,75 | 0,85 | 14 |

Les pastilles conformes à l'invention présentent une masse volumique avant et après compression (respectivement $M_{avant}$ et $M_{après}$) très supérieures à celles des produits existants sur le marché (écailles et cristaux). Lesdites pastilles présentent en outre une compressibilité nettement inférieure à celle des écailles et des cristaux.

Par rapport aux autres modes de mise en forme, les pastilles d'isosorbide sont donc plus aptes à être transportées en gros volume puisqu'elles sont moins compressibles.

Exemple 3 : Comparaison du comportement des compositions d'isosorbide conformes à l'invention et des compositions d'isosorbide sous forme d'écailles ou de cristaux lors de tests de mottage

L'aptitude au mottage de pastilles conformes à l'invention, d'écailles et de cristaux d'isosorbide a été évaluée selon le test de mottage C tel que décrit précédemment.

Les résultats de cette évaluation sont présentés dans le tableau 3.

Tableau 3 : Résultats des tests de mottage en étuve ventilée et thermostatée à 20°C ou à 40°C effectués sur différentes mises en forme d'isosorbide

**Etuve thermostatée à 20°C:**

| Temps (semaines) | Pastilles | | | Cristaux | | Ecailles |
|---|---|---|---|---|---|---|
| | V1 | V2 | V3 | Vrac | 355-1400μm | |
| 2 | 1 | 1 | 1 | 2 | 2 | 1 |
| 4 | 1 | 1 | 1 | 2 | 2 | 1 |

**Etuve thermostatée à 40°C:**

| Temps (semaines) | Pastilles | | | Cristaux | | Ecailles |
|---|---|---|---|---|---|---|
| | V1 | V2 | V3 | Vrac | 355-1400μm | |
| 2 | 2 | 1 | 1 | 2 | 2 | 2 |
| 4 | 2 | 2 | 2 | 3 | 3 | 2 |

Scores : 0 = produit s'écoulant complètement et immédiatement quand le flacon est placé à 90° ; 1 = produit s'écoulant complètement dans une durée maximale de 1 minute quand le flacon est placé à 180° ; 2 = produit ne s'écoulant pas, même après que le flacon ait été placé à 180° durant plus d'une minute, mais s'écoulant après que l'on ait fait chuter, sur le culot du flacon placé à 180°C, une masse de 300g d'une hauteur de 10 cm ; 3 = produit s'il ne s'écoulant pas, même après que le flacon ait été placé à 180° durant plus d'une minute et que l'on ait fait chuter, sur le culot du flacon placé à 180°C, une masse de 300g d'une hauteur de 10 cm.

Les pastilles d'isosorbide conformes à l'invention présentent un score de 1 au test de mottage C réalisé à 20°C durant 2 ou 4 semaines et un score de 1 ou 2 au test de mottage C réalisé à 40°C durant 2 ou 4 semaines.

Exemple 4 : Analyse de la stabilité chimique de pastilles d'isosorbide conformes à l'invention

La stabilité au stockage de pastilles obtenues selon l'exemple 1 a été évaluée selon le test D tel que décrit précédemment.

Les résultats de cette évaluation sont présentés dans le tableau 4.

Tableau 4 : Résultats des tests de stabilité D effectués sur les pastilles conformes à l'invention

| Durée de stockage | Pastilles V1 | Pastilles V2 | Pastilles V3 |
|---|---|---|---|
| 0 jour | 7,6 | 7,6 | 7,6 |
| 15 jours | 7,9 | 7,8 | 8,0 |
| 1 mois | 8,0 | 7,6 | 8,0 |
| 2 mois | 8,1 | 7,8 | 7,9 |
| 3 mois | 8,0 | 7,6 | 8,1 |
| 4 mois | 7,9 | 7,4 | 8,0 |
| 6 mois | 8,1 | 7,8 | 8,1 |

Le pH des différentes pastilles V1, V2, V3 reste stable même après 6 mois de conservation à 50°C, ce qui montre une absence de génération d'acidité (synonyme de dégradation de l'isosorbide avec formation d'acide formique) et ainsi une excellente stabilité des pastilles d'isosorbide conformes à l'invention.

Exemple 5 : Evaluation des durées de solubilisation et de fusion de pastilles d'isosorbide conformes à l'invention et des compositions d'isosorbide sous forme d'écailles ou de cristaux

La durée de solubilisation et celle de fusion de différentes mises en forme d'isosorbide ont été évaluées selon les tests E et F respectivement.

Les résultats de cette évaluation sont présentés dans les tableaux 5 et 6 respectivement.

Tableau 5 : Résultats des tests de solubilisation E effectués sur différentes mises en forme d'isosorbide

| Mise en forme (100 g) | Temps de solubilisation dans l'eau (min) | |
|---|---|---|
| | 20°C | 40°C |
| Ecailles | 5,7 | 4,1 |
| Cristaux Vrac | 8,9 | 3,6 |
| Pastilles V1 | 6,6 | 3,4 |
| Pastilles V2 | 7,8 | 3,6 |
| Pastilles V3 | 9,9 | 4,9 |

Tableau 6 : Résultats des tests de fusion F effectués sur différentes mises en forme d'isosorbide

| Mise en forme | Temps de fusion (min) |
|---|---|
| Ecailles | 15,9 |
| Cristaux Vrac | 17,8 |
| Pastilles V1 | 16,2 |
| Pastilles V2 | 20,4 |
| Pastilles V3 | 22,8 |

Les pastilles d'isosorbide conformes à l'invention présentent une durée de solubilisation sensiblement la même que celles des autres mises en forme connues jusqu'alors.

Lesdites pastilles présentent, en outre, une durée de fusion du même ordre de grandeur que celle des autres mises en forme connues jusqu'alors.

Exemple 6 : Analyse granulométrique des compositions d'isosorbide conformes à l'invention et des compositions d'isosorbide sous forme d'écailles ou de cristaux

La granulométrie sur tamis de différentes mises en forme d'isosorbide a été évaluée selon le test G. Les résultats de cette évaluation sont présentés dans le tableau 7.

Tableau 7 : Granulométrie selon le test G effectuée sur différentes mises en forme d'isosorbide (% de refus en poids)

| Mise en forme | Granulométrie sur tamis (µm) | | | | | | | |
|---|---|---|---|---|---|---|---|---|
| | >20000 | 5000 à 20000 | 2000 à 5000 | 1400 à 2000 | 1000 à 1400 | 500 à 1000 | 355 à 500 | <355 |
| Pastilles V1 | 0 | 51,2 | 46,2 | 1,4 | 1,2 | 0,1 | 0 | 0 |
| Pastilles V2 | 0 | 64,0 | 31,9 | 2,5 | 1,4 | 0,3 | 0 | 0 |
| Pastilles V3 | 0 | 63,0 | 31,1 | 3,8 | 1,1 | 0,8 | 0,2 | 0 |
| Ecailles | 0 | 20,3 | 52,1 | 14,6 | 10,5 | 2,6 | 0 | 0 |
| Cristaux Vrac | 0 | 0,9 | 6,2 | 9,1 | 71,2 | 12,6 | 0 | 0 |
| Cristaux 355 - 1400µm | 0 | 0,1 | 0,3 | 8,4 | 87, 7 | 3,3 | 0,2 | 0 |

Les pastilles d'isosorbide conformes à l'invention sont également caractérisées en ce qu'au moins 90%, préférentiellement 94% en poids, des pastilles présentent une granulométrie sur tamis supérieure à 2000 µm, préférentiellement comprise entre 2000 µm et 20000 µm.

**Revendications**

**1.** Pastilles de dianhydrohexitols de grande fluidité et non mottantes comprenant entre 90% et 100% de dianhydro-hexitols en poids sur sec et dont au moins 90% en poids desdites pastilles présentent une granulométrie sur tamis supérieure ou égale à 2000 µm.

**2.** Pastilles de dianhydrohexitols selon la revendication 1 comprenant moins de 2% d'agent anti-mottant en poids sur sec.

**3.** Pastilles de dianhydrohexitols selon la revendication 1 ou 2, **caractérisées en ce que** lesdits dianhydrohexitols sont choisis parmi l'isosorbide, l'isomannide, l'isoidide et les mélanges d'au moins deux de ces produits.

**4.** Pastilles de dianhydrohexitols selon l'une quelconque des revendications 1 à 3, **caractérisées en ce qu'**elles présentent

- une masse volumique aérée comprise entre 0,80 et 1,00 g/ml,
- une masse volumique tassée, évaluée selon un test A, comprise entre 0,81 et 1,00 g/ml.

**5.** Pastilles de dianhydrohexitols selon l'une quelconque des revendications 1 à 4, **caractérisées en ce qu'**elles présentent un tassement, évalué selon le test A, au plus égal à 2%.

**6.** Pastilles de dianhydrohexitols selon l'une quelconque des revendications 1 à 5, **caractérisées en ce qu'**elles présentent une compressibilité, évaluée selon un test B réalisé à 20°C, inférieure à 5%.

**7.** Pastilles de dianhydrohexitols selon l'une quelconque des revendications 1 à 6, **caractérisées en ce qu'**elles présentent un score de 1 à un test de mottage C réalisé à 20°C durant 2 ou 4 semaines et un score de 1 ou 2 au test de mottage C réalisé à 40°C durant 2 ou 4 semaines.

**8.** Pastilles de dianhydrohexitols selon l'une quelconque des revendications 1 à 7, **caractérisées en ce qu'**elles présentent un pH supérieur ou égal à 7 après 6 mois de stockage dans une étuve ventilée, thermostatée à une température de 50°C.

9. Pastilles de dianhydrohexitols selon l'une quelconque des revendications 1 à 8, **caractérisées en ce qu'**elles présentent une teneur en humidité inférieure à 1% en poids.

10. Pastilles de dianhydrohexitols selon l'une quelconque des revendications 1 à 9, **caractérisées en ce qu'**elles présentent une face plane et une face convexe.

11. Procédé de préparation de pastilles de dianhydrohexitols selon l'une quelconque des revendications 1 à 10, **caractérisé en ce qu'**il comprend les étapes suivantes:

    - production de gouttes d'un fondu de dianhydrohexitols;
    puis
    - refroidissement desdites gouttes.

12. Procédé de préparation de pastilles de dianhydrohexitols selon la revendication 11, **caractérisé en ce que** l'étape de production de gouttes est réalisée en injectant, par surpression, le fondu dans un ou plusieurs générateurs de gouttes.

13. Procédé de préparation de pastilles de dianhydrohexitols selon la revendication 11 ou 12, **caractérisé en ce que** l'étape de refroidissement des gouttes est réalisée par trempe ou par dépôt des gouttes sur une surface métallique en mouvement refroidie par un liquide réfrigérant.

14. Utilisation des pastilles de dianhydrohexitols selon l'une quelconque des revendications 1 à 10 ou obtenues par le procédé selon l'une quelconque des revendications 11 à 13 pour la fabrication de dérivés de dianhydrohexitols et de polymères contenant au moins un dianhydrohexitol ou un dérivé de celui-ci .

**Patentansprüche**

1. Pastillen aus Dianhydrohexitolen hoher Fließfähigkeit und nicht-verklumpend, umfassend zwischen 90 und 100 Trockengewichts-% von Dianhydrohexitolen, und von denen mindestens 90 Gewichts-% der Pastillen eine Partikelgröße auf dem Sieb größer oder gleich 2000 $\mu$m aufweisen.

2. Pastillen aus Dianhydrohexitolen nach Anspruch 1, umfassend mindestens 2 Trockengewichts-% des Anti-Verklumpungsmittels.

3. Pastillen aus Dianhydrohexitolen nach Anspruch 1 oder 2, **dadurch gekennzeichnet, dass** die Dianhydrohexitole ausgewählt sind aus Isosorbid, Isomannid, Isoidid und Mischungen von wenigstens zwei dieser Produkte.

4. Pastillen aus Dianhydrohexitolen nach einem der Ansprüche 1 bis 3, **dadurch gekennzeichnet, dass** sie aufweisen

    - eine Schüttdichte zwischen 0,80 und 1,00 g/ml
    - eine Klopfdichte zwischen 0,81 und 1,00 g/ml, welche nach dem Test A bestimmt ist.

5. Pastillen aus Dianhydrohexitolen nach einem der Ansprüche 1 bis 4, **dadurch gekennzeichnet, dass** sie eine Verdichtung höchstens gleich 2% aufweisen, welche nach dem Test A bestimmt ist.

6. Pastillen aus Dianhydrohexitolen nach einem der Ansprüche 1 bis 5, **dadurch gekennzeichnet, dass** sie eine Kompressibilität von weniger als 5% aufweisen, welche nach einem Test B bestimmt ist, welcher bei 20°C durchgeführt wird.

7. Pastillen aus Dianhydrohexitolen nach einem der Ansprüche 1 bis 6, **dadurch gekennzeichnet, dass** sie einen Wert von 1 bei einem Verklumpungstest C, welcher bei 20 °C während 2 oder 4 Wochen durchgeführt wird, und einen Wert von 1 oder 2 bei dem Verklumpungstest C, welcher bei 40 °C während 2 oder 4 Wochen durchgeführt wird, aufweisen.

8. Pastillen aus Dianhydrohexitolen nach einem der Ansprüche 1 bis 7, **dadurch gekennzeichnet, dass** sie einen pH-Wert höher als oder gleich 7 nach 6 Monaten Lagerung in einem belüfteten, bei einer Temperatur von 50 °C thermostatisierten Trockenschrank aufweisen.

**9.** Pastillen aus Dianhydrohexitolen nach einem der Ansprüche 1 bis 8, **dadurch gekennzeichnet, dass** sie einen Feuchtigkeitsgehalt weniger als 1 Gewichts-% aufweisen.

**10.** Pastillen aus Dianhydrohexitolen nach einem der Ansprüche 1 bis 9, **dadurch gekennzeichnet, dass** sie eine flache Seite und eine konvexe Seite aufweisen.

**11.** Verfahren zur Herstellung von Pastillen aus Dianhydrohexitolen nach einem der Ansprüche 1 bis 10, **dadurch gekennzeichnet, dass** es die folgenden Schritte umfasst:

   - Herstellen von Tropfen aus einer Schmelze aus Dianhydrohexitolen; dann
   - Abkühlen der Tropfen.

**12.** Verfahren zur Herstellung von Pastillen aus Dianhydrohexitolen nach Anspruch 11, **dadurch gekennzeichnet, dass** der Schritt zum Herstellen der Tropfen durch Einspritzen der Schmelze bei Überdruck in einen oder mehrere Tropfengeneratoren durchgeführt wird.

**13.** Verfahren zur Herstellung von Pastillen aus Dianhydrohexitolen nach Anspruch 11 oder 12, **dadurch gekennzeichnet, dass** der Schritt zum Abkühlen der Tropfen durch Abschrecken oder durch Aufbringen der Tropfen auf eine bewegliche, durch eine Kühlflüssigkeit gekühlte Metalloberfläche durchgeführt wird.

**14.** Verwendung der Pastillen aus Dianhydrohexitolen nach einem der Ansprüche 1 bis 10 oder erhalten durch das Verfahren nach einem der Ansprüche 11 bis 13 bei der Herstellung von Derivaten aus Dianhydrohexitolen und von Polymeren enthaltend mindestens ein Dianhydrohexitol oder ein Derivat davon.

**Claims**

**1.** High-fluidity and non-caking dianhydrohexitol pellets comprising between 90% and 100% by weight of dianhydrohexitols on a dry basis and in which at least 90% by weight of the pellets exhibit a particle size on sieves of greater than or equal to 2000 $\mu$m.

**2.** The dianhydrohexitol pellets as claimed in claim 1, comprising less than 2% by weight of anticaking agent on a dry basis.

**3.** The dianhydrohexitol pellets as claimed in claim 1 or 2, **characterized in that** said dianhydrohexitols are chosen from isosorbide, isomannide, isoidide and the mixtures of at least two of these products.

**4.** The dianhydrohexitol pellets as claimed in any one of claims 1 to 3, **characterized in that** they exhibit:

   - a bulk density of between 0.80 and 1.00 g/ml,
   - a tapped density, evaluated according to a test A, of between 0.81 and 1.00 g/ml.

**5.** The dianhydrohexitol pellets as claimed in any one of claims 1 to 4, **characterized in that** they exhibit a tapping, evaluated according to the test A, at most equal to 2%.

**6.** The dianhydrohexitol pellets as claimed in any one of claims 1 to 5, **characterized in that** they exhibit a compressibility, evaluated according to a test B carried out at 20°C, of less than 5%.

**7.** The dianhydrohexitol pellets as claimed in any one of claims 1 to 6, **characterized in that** they exhibit a score of 1 in a caking test C carried out at 20°C for 2 or 4 weeks and a score of 1 or 2 in the caking test C carried out at 40°C for 2 or 4 weeks.

**8.** The dianhydrohexitol pellets as claimed in any one of claims 1 to 7, **characterized in that** they exhibit a pH of greater than or equal to 7 after 6 months of storage in a ventilated oven thermostatically controlled at a temperature of 50°C.

**9.** The dianhydrohexitol pellets as claimed in any one of claims 1 to 8, **characterized in that** they exhibit a moisture content of less than 1% by weight.

10. The dianhydrohexitol pellets as claimed in any one of claims 1 to 9, **characterized in that** they exhibit a flat face and a convex face.

11. A process for the preparation of dianhydrohexitol pellets as claimed in any one of claims 1 to 10, **characterized in that** it comprises the following stages:

   - production of drops of a dianhydrohexitol melt; then
   - cooling of said drops.

12. The process for the preparation of dianhydrohexitol pellets as claimed in claim 11, **characterized in that** the stage of production of drops is carried out by injecting, by excess pressure, the melt into one or more drop generators.

13. The process for the preparation of dianhydrohexitol pellets as claimed in claim 11 or 12, **characterized in that** the stage of cooling of the drops is carried out by quenching or by dropping the drops onto a moving metal surface cooled by a cooling liquid.

14. The use of the dianhydrohexitol pellets as claimed in any one of claims 1 to 10 or obtained by the process as claimed in any one of claims 11 to 13 in the manufacture of derivatives of dianhydrohexitols and of polymers comprising at least one dianhydrohexitol or one derivative of the latter.

**EP 2 621 930 B1**

## RÉFÉRENCES CITÉES DANS LA DESCRIPTION

**Documents brevets cités dans la description**

- JP 7488183 B
- FR 2477902
- JP 2006117649 A
- FR 2919587
- EP 1446373 A
- EP 1287000 A
- WO 2009010673 A
- EP 1787993 A1